Europäisches Patentamt

European Patent Office

Office européen des brevets

(19)

(11) Publication number : **0 502 718 A1**

(12) **EUROPEAN PATENT APPLICATION**

(21) Application number : **92301868.3**

(22) Date of filing : **04.03.92**

(51) Int. Cl.⁵ : **A01N 37/46,** A01N 37/44, A01N 63/00, A01N 65/00, C12N 15/00, C12N 15/52

(30) Priority : **04.03.91 US 664270**

(43) Date of publication of application :
**09.09.92 Bulletin 92/37**

(84) Designated Contracting States :
**AT BE CH DE DK ES FR GB GR IT LI LU NL SE**

(71) Applicant : **PIONEER HI-BRED INTERNATIONAL, INC.**
**700 Capital Square 400 Locust Street**
**Des Moines Iowa 50309 (US)**

(72) Inventor : **Duvick, Jonathan**
**1707 38th Street, Des Moines**
**Polk County, Iowa 50310 (US)**
Inventor : **Rood, Tracy**
**4333 Allison Avenue, Des Moines**
**Polk County, Iowa 50310 (US)**

(74) Representative : **Goldin, Douglas Michael et al**
**J.A. KEMP & CO. 14, South Square Gray's Inn**
**London WC1R 5LX (GB)**

(54) **Natural and synthetic proteins with inhibitory activity towards pathogenic microorganisms.**

(57) A selected group of proteins has been found to have antimicrobial properties. In a preferred embodiment, plant resistance to diseases caused by plant pathogens which are susceptible to these proteins is produced by inserting into the cells of a plant a gene whose expression causes production a selected protein in antimicrobial amounts.

EP 0 502 718 A1

## Technical Field

This invention relates to materials and methods for killing and inhibiting fungal and other pathogens of plants, humans and animals, and materials and methods for directly imparting disease resistance to plants.

## Background of the Invention

There is a great interest in the development of new microbicides that do not have adverse effects on the host organism but have high potency against pathogens. One area of interest in locating microbicidally active compounds is naturally occurring compounds, because they can be readily detoxified by natural mechanisms in the environment and in humans and other animals.

## Humans and Lower Animals

Bacteria and fungi are the cause of many diseases in humans and lower animals. These diseases can range from minor irritations such as athlete's foot to serious or even fatal systemic infections. Often the medications used in treating systemic infections, particularly those used in treating systemic fungal infections, cause serious side affects in the treated human or animal. Therefore there is a continuing need in the fields of human and veterinary medicine for the development of new and effective antiinfective compounds.

## Plants

Numerous fungi and bacteria are not only pathogens in humans and lower animals, but are also serious pests of agricultural crops. One method of controlling plant diseases in the past has been to apply antimicrobial organic or in the past has been to apply antimicrobial organic or semiorganic chemicals to crops. This method has numerous, art-recognized problems. A more recent method of control of microorganism pests has been the use of biological control organisms which are typically natural competitors or inhibitors of the troublesome microorganisms. However, it is difficult to apply biolgical control organisms to large areas, and even more difficult to cause those living organisms to remain in the treated area for an extended period. Still more recently, techniques in recombinant DNA have provided the opportunity to insert into plant cells cloned genes which express proteins in the plant cells. Some proteins are known to have antimicrobial activity. However, this technology has given rise to additional concerns about eventual microbial resistance to well-known, naturally occurring antimicrobials, particularly in the face of heavy selection pressure, which may occur in some areas. Thus, a continuing need exists to identify additional naturally occurring antimicrobial compounds which can be formed by plant cells directly by translation of a single structural gene.

European Patent Application 204,590, based upon U.S. Patent application Serial No. 725,368, describes a method of genetically modifying a plant cell to control expression of heterologous foreign structure genes. In the method, the plant cell is transformed to contain a pRi T-DNA promoter and a heterologous foreign structural gene, the promoter and the structural gene being in such position and orientation with respect to one another that the structural gene is expressible in a plant cell under control of the promoter.

Likewise, European Patent Application 186,425, based upon U.S. patent application Serial No. 685,824, describes a recombinant DNA expression vector which comprises (a) a transcription unit, flanked by T-DNA border sequences, which comprises a promoter and associated amino terminal region encoding sequences and a terminator signal sequence in which the sequences are derived from one or more genes which are naturally expressed in a plant cell, and (b) an antibiotic resistance gene-encoding sequence located between the promoter and associated amino-terminal region-encoding sequence and the terminator sequence and (c) a DNA fragment containing a replicon that is functional in Agrobacterium.

PCT application 8807087, based upon U.S. patent application 168,109, discloses a recombinant virus expression system comprising a Heliothis polyhedin promoter and a nucleotide sequence encoding a heterologous peptide or protein, which may have antimicrobial activity.

## Summary of the Invention

According to the invention, certain natural and synthetic proteins have been determined to have potent antimicrobial activity against many common pathogens. This invention thus relates broadly to compositions containing these proteins, and methods of using these proteins and compositions containing them against human, animal and plant pathogens. Since it is presently considered feasible and desirable to genetically engineer plants to express proteins which, inter alia, impart resistances to diseases and other pests, this invention also

EP 0 502 718 A1

relates broadly to methods of creating plants with disease resistance by virtue of expressing one or more of these proteins, and to cells and plants created thereby.

## Disclosure of the Invention

It has now been determined that proteins from the group consisting of:

```
Defensin NP1              poly-L-Arginine HCl
Magainin-2                poly-L-Histidine
Magainin-A                Adrenocorticotropic Hormone 1-17
Magainin-G                Adrenocorticotropic Hormone 1-24
α-Hordothionin            Cathepsin G
β-Purothionin             β-Hordothionin
Melittin                  Eosinophil Major Basic Protein
poly-L-Lysine HBr         Eosinophil Cationic Protein
```

```
        poly-L-Lysine HCl        Eosinophil Peroxidase
        poly-D-Lysine            Crotamine
        poly-D-Lysine HBr        Citrate Synthase
        Mastoparan               Stinging Nettle Lectin
        Kassinin                 Lyticase
        Cytohelicase             Penicillinase
        Lysozyme
```

Pro-His-Pro-Phe-His-Phe-Phe-Val-Tyr-Lys and mixtures thereof have potent antimicrobial activity against many common pathogens. These proteins are particularly active against the following group of pathogens: Fusarium graminearum, Fusarium moniliforme, Diplodia maydis, Colletototrichum graminicola, Verticillium albo-atrum, Phytophthora megaspermae f.sp. glycinea, Macrophomina phaseolina, Diaporthe phaseolorum caulivora, Sclerotinia sclerotiorum, Sclerotinia trifoliorum, Aspergillus flavus and Alternaria longipes.

Thus, this invention provides a method for killing or inhibitory susceptible pathogens, including microorganisms selected from the group listed above, comprising the step of introducing into the environment of the organisms an antimicrobial amount of a protein selected from the group consisting of:

3

| | |
|---|---|
| Defensin NP1 | poly-L-Arginine HCl |
| Magainin-2 | poly-L-Histidine |
| Magainin-A | Adrenocorticotropic Hormone 1-17 |
| Magainin-G | Adrenocorticotropic Hormone 1-24 |
| α-Hordothionin | Cathepsin G |
| β-Purothionin | β-Hordothionin |
| Melittin | Eosinophil Major Basic Protein |
| poly-L-Lysine HBr | Eosinophil Cationic Protein |
| poly-L-Lysine HCl | Eosinophil Peroxidase |
| poly-D-Lysine | Crotamine |
| poly-D-Lysine HBr | Citrate Synthase |
| Mastoparan | Stinging Nettle Lectin |

| | |
|---|---|
| Kassinin | Lyticase |
| Cytohelicase | Penicillinase |
| Lysozyme | |

Pro-His-Pro-Phe-His-Phe-Phe-Val-Tyr-Lys and mixtures thereof.

This invention also provides compositions of matter for the treatment and prevention of diseases in humans, lower animals, and plants caused by susceptible organisms, comprising one or both of the foregoing polypeptides in combination with a non-toxic carrier. The terms "protein" and "polypeptide" are used herein interchangeably as applied to the specific compounds identified above.

**Plants**

The polypeptides employed in this invention can be effectively applied to plants afflicted with susceptible microorganisms by any convenient means, including spray, creams, dust or other formulation common to the antimicrobial arts. The polypeptide can also be incorporated systemically into the tissues of a treated plant so that in the course of infesting the plant the pathogens will be exposed to antimicrobial amounts of the antimicrobial protein. One method of doing this is to incorporate the protein in a non-phytotoxic vehicle which is adapted for systemic administration to the susceptible plants. This method is commonly employed with fungicidal materials such as captan and is well within the purview of one of ordinary skill in the art of plant fungicide formulation. However, since the genes which code for these proteins can be isolated, cloned, inserted into an appropriate expression cassette, and introduced into cells of a susceptible plant species, an especially preferred embodiment of this method involves inserting into the genome of the plant a DNA sequence coding for one or more of the foregoing polypeptides in proper reading frame, together with transcription initiator and promoter sequences active in the plant. Transcription and translation of the DNA sequence under control of the regulatory sequences causes expression of the protein or proteins at antimicrobial levels in the tissues of the plant which are normally infected by the pathogens.

The plant is preferably a plant susceptible to infection and damage by one or more of Fusarium graminearum, Fusarium moniliforme, Aspergillus flavus, Alternaria longipes, Sclerotinia sclerotiorum, and Sclerotinia trifoliorum. These include corn (Zea mays) and sorghum (Sorghum bicolor). However, this is not to be construed as limiting, inasmuch as these two species are among the most difficult commercial crops to reliably transform and regenerate, and these pathogens also infect certain other crops. Thus the methods of this invention are readily applicable via conventional techniques to numerous plant species, if they are found to be susceptible to the plant pathogens listed hereinabove, including, without limitation, species from the genera Fragaria, Lotus, Medicago, Onobrychis, Trifolium, Trigonella, Vigna, Citrus, Linum, Geranium, Manicot, Daucus, Arabidopsis, Brassica, Raphanus, Sinapis, Atropa, Capsicum, Datura, Hyoscyamus, Lycopersionn, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Triticum, and Datura.

Preferred plants that are to be transformed according to the methods of this invention are cereal crops, including maize, rye, barley, wheat, sorghum, oats, millet, rice, triticale, sunflower, alfalfa, rape seed and soybean.

The DNA sequence which when expressed imparts antimicrobial activity is a structural gene which codes for the selected protein or proteins as described herein. In general, since the object of the invention is to confer resistance to a microorganism to which the plant is susceptible, in many cases the protein will not be native to the plant, i.e., the protein and the gene for the protein will be synthetic or will come from a species other than the plant being transformed. However, in species which produce one or more of these proteins but in lower than antimicrobial amounts, it may be preferable to insert a gene for the selected protein or proteins under strong constitutive promoter control to cause overproduction of the protein, thus achieving antimicrobial levels and conferring effective disease resistance. Alternatively, where a plant produces the one or more of the foregoing proteins but the protein or proteins is not produced in or distributed to tissues which are normally affected by the disease, a tissue-specific promoter can be used to provide localized expression or overproduction of the protein. A tissue-specific promoter can be used in any instance where it may be desirable to localize production of the protein to an tissue which is susceptible to infection or to a tissue which is efficient in production of the protein.

The DNA sequences which code for the selected protein in the practice of this invention can be obtained from natural sources of the protein by conventional techniques and the gene can then be removed by use of appropriate restriction enzymes and spliced into a selected plant expression cassette. Alternatively, a purified protein can be sequenced in its entirety using known methods, and synthetic DNA sequences can then be prepared which code for the appropriate sequence of amino acids, and this synthetic sequence can be inserted into an appropriate plant expression cassette.

Likewise, numerous plant expression cassettes and vectors are well known in the art. By the term "expression cassette" is meant a complete set of control sequences including initiation, promoter and termination sequences which function in a plant cell when they flank a structural gene in the proper reading frame. Expression cassettes frequently and preferably contain an assortment of restriction sites suitable for cleavage and insertion of any desired structural gene. It is important that the cloned gene have a start codon in the correct reading frame for the structural sequence. In addition, the plant expression cassette preferably includes a strong constitutive promoter sequence at one end to cause the gene to be transcribed at a high frequency, and a poly-A recognition sequence at the other end for proper processing and transport of the messenger RNA. An example of such a preferred (empty) expression cassette into which the cDNA of the present invention can be inserted is the pPHI414 plasmid developed by Beach et al. of Pioneer Hi-Bred International, Inc., Johnston, IA. Highly preferred plant expression cassettes will be designed to include one or more selectable marker genes, such as kanamycin resistance or herbicide tolerance genes.

By the term "vector" herein is meant a DNA sequence which is able to replicate and express a foreign gene in a host cell. Typically, the vector has one or more endonuclease recognition sites which may be cut in a predictable fashion by use of the appropriate enzyme. Such vectors are preferably constructed to include additional structural gene sequences imparting antibiotic or herbicide resistance, which then serve as markers to identify and separate transformed cells. Preferred markers/selection agents include kanamycin, chlorosulfuron, phosphonothricin, hygromycin and methotrexate. A cell in which the foreign genetic material in a vector is functionally expressed has been "transformed" by the vector and is referred to as a "transformant."

A particularly preferred vector is a plasmid, by which is meant a circular double-stranded DNA molecule which is not a part of the chromosomes of the cell.

Promoters that may be used in the genetic sequence include nos, ocs and CaMV promoters.

An efficient plant promoter that may be used is an overproducing plant promoter. Overproducing plant promoters that may be used in this invention include the promoter of the small sub-unit (ss) of the ribulose-1,5-biphosphate carboxylase from soybean (Berry-Lowe et al, J. Molecular and App. Gen., 1:483-498 (1982)), and the promoter of the cholorophyll a-b binding protein. These two promoters are known to be light-induced in eukaryotic plant cells (see, for example, Genetic Engineering of Plants, An Agricultural Perspective, A. Cashmore, Pelham, New York, 1983, pp. 29-38, G. Coruzzi et al., J. Biol. Chem., 258:1399 (1983), and P. Dunsmuir, et al., J. Molecular and App. Gen., 2:285 (1983)).

The expression cassette comprising the structural gene for a selected protein operably linked to the desired control sequences can be ligated into a suitable cloning vector. In general, plasmid or viral (bacteriophage) vectors containing replication and control sequences derived from species compatible with the host cell are used. The cloning vector will typically carry a replication origin, as well as specific genes that are capable of providing phenotypic selection markers in transformed host cells. Typically, genes conferring resistance to antibiotics or selected herbicides are used. After the genetic material is introduced into the target cells, successfully transformed cells and/or colonies of cells can be isolated by selection on the basis of these markers.

Typically, an intermediate host cell will be used in the practice of this invention to increase the copy number of the cloning vector. With an increased copy number, the vector containing the gene of interest can be isolated in significant quantities for introduction into the desired plant cells. Host cells that can be used in the practice of this invention include prokaryotes, including bacterial hosts such as E. coli, S. typhimurium, and Serratia marcescens. Eukaryotic hosts such as yeast or filamentous fungi may also be used in this invention. Since these hosts are also microorganisms, it will be essential to ensure that plant promoters which do not cause expression of the selected protein in bacteria are used in the vector.

The isolated cloning vector will then be introduced into the plant cell using any convenient technique, including electroporation (in protoplasts), Agrobacterium species, retroviruses, microparticle bombardment, and microinjection, into cells from monocotyledonous or dicotyledonous plants, in cell or tissue culture, to provide transformed plant cells containing as foreign DNA at least one copy of the DNA sequence of the plant expression cassette. Preferably, the monocotyledonous species will be selected from maize, sorghum, wheat and rice, and the dicotyledonous species will be selected from soybean, alfalfa, tobacco, rapeseed and tomato. Using known techniques, protoplasts can be regenerated and cell or tissue cultures can be regenerated to form whole fertile plants which carry and express the gene for the selected protein. Accordingly, a highly preferred embodiment of the present invention is a transformed maize plant, the cells of which contain as foreign DNA at least one copy of the DNA sequence of an expression cassette of this invention.

Finally, this invention provides methods of imparting resistance to diseases caused by microorganisms selected from Fusarium graminearum, Fusarium moniliforme, Diplodia maydis, Colletototrichum graminicola, Verticillium albo-atrum, Phytophthora megaspermae f.sp. glycinea, Macrophomina phaseolina, Diaporthe phaseolorum caulivora, Sclerotinia sclerotiorum, Sclerotinia trifoliorum, Aspergillus flavus and Alternaria longipes to plants of a susceptible taxon, comprising the steps of:

a) culturing regenerable cells or tissues from at least one plant from the taxon,

b) introducing into the cells of the cell or tissue culture at least one copy of an expression cassette comprising a structural gene for a selected protein, operably linked to plant regulatory sequences which cause the expression of the structural gene for the selected protein in the cells, and

c) regenerating disease-resistant whole plants from the cell or tissue culture. Once whole plants have been obtained, they can be sexually or clonally reproduced in such manner that at least one copy of the sequence provided by the expression cassette is present in the cells of progeny of the reproduction.

Alternatively, once a single transformed plant has been obtained by the foregoing recombinant DNA method, conventional plant breeding methods can be used to transfer the structural gene for the selected protein and associated regulatory sequences via crossing and backcrossing. Such intermediate methods will comprise the further steps of

a) sexually crossing the disease-resistant plant with a plant from the disease-susceptible taxon;

b) recovering reproductive material from the progeny of the cross; and

c) growing disease-resistant plants from the reproductive material. Where desirable or necessary, the agronomic characteristics of the susceptible taxon can be substantially preserved by expanding this method to include the further steps of repetitively:

a) backcrossing the disease-resistant progeny with disease-susceptible plants from the susceptible taxon; and

b) selecting for expression of antimicrobial activity (or an associated marker gene) among the progeny of the backcross, until the desired percentage of the characteristics of the susceptible taxon are present in the progeny along with the gene imparting antimicrobial activity.

By the term "taxon" herein is meant a unit of botanical classification of genus or lower. It thus includes genus, species, cultivars, varieties, variants, and other minor taxonomic groups which lack a consistent nomenclature.

It will also be appreciated by those of ordinary skill that the plant vectors provided herein can be incorporated into Agrobacterium tumefaciens, which can then be used to transfer the vector into susceptible plant cells, especially those from dicotyledonous species. Thus, this invention provides a method for imparting antimicrobial activity and disease resistance in Agrobacterium tumefaciens-susceptible dicotyledonous plants in which the expression cassette is introduced into the cells by infecting the cells with Agrobacterium tumefaciens, a plasmid of which has been modified to include a plant expression cassette of this invention.

## Human and Veterinary Pharmaceutical Use

This invention also provides methods of treating and preventing infection by susceptible organisms in a human or lower animal host in need of such treatment, which method comprises administration to the human or lower animal host in need of such treatment a therapeutically effective amount of a polypeptide of this invention or a composition containing one or both of the polypeptides. The polypeptides of the present invention may be administered parenterally, by inhalation spray, rectally or topically in dosage unit formulations containing conventional nontoxic pharmaceutically acceptable carriers, adjuvants and vehicles as desired. The term parenteral as used herein includes subcutaneous, intravenous, intramuscular, intraarticular and intrathecal injection and infusion techniques. As with other polypeptides, the polypeptides of this invention are not known to be active orally.

Total daily dose of the compounds of this invention administered to a host in single or divided doses may be in amounts, for example, of from 0.1 to 2000 mg/kg body weight daily and more usually 50 to 500 mg/kg. Dosage unit compositions may contain such amounts or fractions or submultiples thereof as appropriate to make up the daily dose. It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

This invention also provides pharmaceutical compositions in unit dosage form, comprising an effective amount of a compound of this invention in combination with a conventional pharmaceutical carrier. As used herein, the term "pharmaceutical carrier" means a solid or liquid filler, diluent or encapsulating material. Some examples of the materials which can serve as pharmaceutical carriers are sugars, such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such peanut oil, cottonseed oil, safflower oil, sesame oil, olive oil, corn oil and soybean oil; polyols such as propylene glycol, glycerin, sorbitol, mannitol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution, ethyl alcohol and phosphate buffer solutions, as well as other non-toxic compatible substances used in pharmaceutical formulations. Wetting agents, emulsifiers and lubricants such as sodium lauryl sulfate and magnesium stearate, as well as coloring agents, release agents, coating agents, and perfuming agents and preservatives can also be present in the compositions, according to the desires of the formulator. The amount of active ingredient that may be combined with the carrier materials to produce a single dosage form will vary depending upon the host treated and the particular mode of administration.

By "therapeutically effective amount" herein is meant an amount of either polypeptide or combination thereof sufficient to provide antimicrobial activity so as to alleviate or prevent infection by susceptible organisms in the human or lower animal being treated at a reasonable benefit/risk ratio attendant with any medical treatment.

## Industrial Applicability

The following descriptions further exemplify the compositions of this invention and the methods of making and using them. However, it will be understood that other methods, known by those of ordinary skill in the art to be equivalent, can also be employed.

Example 1 - Antimicrobial Screening Procedures

Proteins are screened for antimicrobial activity in concentrations depending on the molecular weight of the selected protein. The following chart gives examples of stock solution concentrations:

| Molecular weight | Stock solution concentration |
|---|---|
| <500 Da | 3000 $\mu$M |
| 500-5000 Da | 300 $\mu$M |
| 5000-10000 Da | 100 $\mu$M |
| >10000 Da | 30 $\mu$M |

Solutions of protein with unknown molecular weight are made with a concentration of 1.0 mg/ml. Water is the usual solvent, however .01% acetic acid may be used for small proteins. The stock solutions are sterilized using means known to the art and are stored at an appropriate temperature.

The proteins are then screened for antimicrobial or antifungal activity by the following methods:

7

Ten µl of a protein solution are put in a well of a microtiter plate. Spores, or hyphal fragments of fungi that do not produce spores, are added to the well. The number of spores per well is approximately 200-250 and the number of hyphal fragments is approximately 10-50.

Plates are incubated at 28°C in the dark for about 20-24 hours. The individual wells are scored visually under an inverted microscope. A qualitative 0-4 scale is used to determine antifungal activity, with 1 being no inhibitionrelative to water control and 4 being complete inhibition. Scores of 1, 2 and 3 indicate intermediate levels of antifungal activity. Tables 1-6 gives the results of the Level 1 screening against 6 pathogenic organisms.

EP 0 502 718 A1

Table 1

## Level I Results of Antifungal Screening

Data represent antifungal scores (0-4 scale) obtained in one or two experiments, two reps per experiment.

| | ALO | AFL | FGR | FMO | SSC | STR |
|---|---|---|---|---|---|---|
| **SYNTHETIC PEPTIDES:** | | | | | | |
| Magainin 2 (30 uM = 75 ug/ml)<br>•GIGKFLHSAKKFGKAFVGEIMNS<br>•from Bachem (PANT 10)<br>•>98% pure, 73.2% peptide<br>•ref: Zasloff, PNAS, 84, Aug 1987, 5449-5453 | 3,3 | 1,0 | 3.5,3.5 | 2,2.5 | 0,0 | 0,0 |
| Magainin A (30 uM = 75 ug/ml)<br>•BAIGKFLHAAKKFAKAFVAEIMNS | 4,4 | .5,1 | 4,4 | 4,4 | 3,3 | 1.5,2 |
| Magainin G (30 uM = 76 ug/ml)<br>•BAGIGKFLHSAKKFAKAFVAEIMNS<br><br>•both from Hao-Chia Chen, NIH<br>•both >97% pure<br>•ref: Chen, FEBS Lett., 236, 2, 462-466 | 4,4 | 1,0 | 4,4 | 4,4 | 0,1 | 0,0 |
| Poly-L-arginine HCl (3 uM = 72 ug/ml)<br>•from Sigma (P7762)<br>•mol wt. 15,000-70,000 (24,000 avg.)<br>•prepared from poly-L-ornithine,<br>contains <3% poly-L-ornithine | 4,4 | 3,3 | 4,4 | 4,4 | 4,4 | 4,4 |
| Poly-L-histidine (3 uM = 38 ug/ml)<br>•from Sigma (P9386)<br>•mol. wt. 5,000-15,000 (12,800 avg.) | 3,3 | .5,0 | 4,4 | 3.5,4 | 2,3 | 3,2.5 |
| Poly-L-histidine (3 uM = 57 ug/ml)<br>•from Sigma (P2534)<br>•mol. wt. 15,000-50,000 (19,000 avg.) | 4,4 | 2,2 | 4,4 | 4,4 | 4,4 | 4,4 |

Table 2

| | ALO | AFL | PGR | FMO | SSC | STR |
|---|---|---|---|---|---|---|
| Mastoparan (30 uM = 44 ug/ml) | 4,4 | 1.5,1.5 | 4,4 | 4,4 | 4,4 | 4,4 |
| •from Sigma (M7517) | 4,4 | 1,1.5 | 4,4 | 4,4 | 4,4 | 3,2 |

•INLKALAALAKKIL
•mast cell degranulating peptide from
wasp venom
•ref: Hirai et al., Chem. Pharm. Bull.,
27, 1942 (1979)

| | ALO | AFL | PGR | FMO | SSC | STR |
|---|---|---|---|---|---|---|
| Kassinin (100 uM = 130 ug/ml) | 0,0 | 1.5,1.5 | 2,0 | 1,0 | 0,0 | 0,0 |
| •DVPKSDQFVFLM | 0,0 | 1,1 | 0,0 | 1.5,1.5 | 0,0 | 0,0 |

•from Sigma (K0876)
•tachykinin peptide with substance P-like activity
•~60% homology to substance P,
which showed no significant activity
•refs: Anastasi et al, Experientia, 33, 857 (1977)
and Guellner, IRCS Med. Sci. Libr. Compend., 9, 685 (1981)

Pro-His-Pro-Phe-His-Phe-Phe-Val-Tyr-Lys
(100 uM = 132 ug/ml)

| | ALO | AFL | PGR | FMO | SSC | STR |
|---|---|---|---|---|---|---|
| •from Sigma (P2402) | 1,0 | 0,0 | 3.5,3 | 1,0 | 0,0 | 0,0 |
| •renin inhibitor | 0,0 | 0,0 | 4,4 | 0,0 | | |

•ref: Burton et al., PNAS, USA, 77, 5476 (1980)

| | ALO | AFL | PGR | FMO | SSC | STR |
|---|---|---|---|---|---|---|
| Adrenocorticotropic hormone | 2.5,0 | 0,0 | 2,2 | 1,1 | 0,0 | 0,0 |
| fragment 1-17 (30 uM = 63 ug/ml) | 0,0 | 0,0 | 2.5,2.5 | 1,1 | 0,0 | |

•SYSMEHFTWGKPVGKKR
•from Sigma (A2407)
•88% peptide, 99% pure
•Chemical Abstract Survey #7266-47-9

| | ALO | AFL | PGR | FMO | SSC | STR |
|---|---|---|---|---|---|---|
| Adrenocorticotropic hormone | 1,1 | 1,1 | 3,3 | 2,1 | 0,0 | 0,1 |
| fragment 1-24 (15 uM = 44 ug/ml) | | | | | | |

•SYSMEHFRWGKPVGKKRRPVKVYP
•from Calbiochem (121721), >98% pure
•N-terminal fragment of the pituitary hormone
that stimulates the secretion of adrenal corticosteroids
and induces growth of the adrenal cortex
•ref: Schwyzer et al. 1961. Helv. Chim. Acta 44, 1136

EP 0 502 718 A1

Table 3

**PURIFIED PEPTIDES, PROTEINS AND ENZYMES**

| | ALO | AFL | FGR | FMO | SSC | STR |
|---|---|---|---|---|---|---|
| Citrate synthase (100 ug/ml) | 1,1 | 0,0 | 3,3 | 1,1 | 0,0 | 0,0 |
| •from chicken heart, chromatographically pure | 0,0 | 0,0 | 3,3 | 1,1 | 0,0 | 0,0 |

•crystalline suspension in 2.2M (NH4)2SO4
solution, pH 7.0 containing a trace of phosphate
•Chemical Abstract Survey #9027-96-7

| | ALO | AFL | FGR | FMO | SSC | STR |
|---|---|---|---|---|---|---|
| Defensin 1 or NP1 (30 uM = 117 ug/ml) | 4,4 | 4,4 | 4,4 | 4,4 | 4,4 | 4,4 |

•VVCACRRALCLPRERRAGFCRIRGRIHPLCCRR
•cationic peptide isolated from rabbit neutrophil cells
•from Robert Lehrer, UCLA
•isolated from rabbit peritoneal granulocytes
•ref: Selsted et al., Infec and Imm, 45, 1, 150-154

| | ALO | AFL | FGR | FMO | SSC | STR |
|---|---|---|---|---|---|---|
| Cathepsin G (3 uM = 70 ug/ml) | 3,3 | 1.5,1 | 4,4 | 4,4 | 1,1 | 1,1 |

•from Athens Research and Technology (16-14-030107)
•isolated from human neutrophil cells
•degrades collagen and proteoglycan
•causes release of angiotensin II from angiotensin I
or angiotensinogen, implicated in connective tissue
diseases (emphysema, rheumatoid arthritis)
•>98% pure
•ref: Travis et al., 1978. Biochem. 17, 5651

| | ALO | AFL | FGR | FMO | SSC | STR |
|---|---|---|---|---|---|---|
| Lysozyme (3 uM = 50 ug/ml) | 1,2 | 0,1 | 4,4 | 3,2 | 1,2 | 2,1 |

•from Athens Research and Technology (16-14-122519)
•isolated from human neutrophil cells
•takes part in antibacterial activity of leukocytes
•hydrolyzes $\beta$-1,4 links between N-acetylmuramic acid
or 2-acetamido-2-deoxy-D-glucose residues in
mucopolysaccharide, mucopolypeptide or chitin
•>98% pure
•ref: Shugar, 1952. Biochim. Biophys. Acta 8, 302

## Table 4

| | ALO | AFL | FGR | FMO | SSC | STR |
|---|---|---|---|---|---|---|
| Alpha hordothionin--Morex (30 uM = 150 ug/ml) •KSCCRSTLGRNCYNLCRVRGAQKLCAGVCRCKLTSSGLCPTGKPK | 4,4 | 3.5,3 | 4,4 | 4,4 | 4,4 | 4,4 |
| Beta hordothionin--Morex (20 uM = 100 ug/ml) •KSCCRSTLGRNCYNLCRVRGAQKLCANACRCKLTSGLKCPSGFPK | 4,4 | 3,3 | 4,4 | 4,4 | 4,4 | 4,4 |
| Beta hordothionin--Bomi (20 uM = 100 ug/ml) | 4,4 | 2,3 | 4,4 | 4,4 | 4,4 | 4,4 |
| Beta hordothionin--Klages (20 uM = 100 ug/ml) •KSCCRSTLGRNCYNLCRVRGAQKLCANACRCKLTSGLKCPSSFPK | 2,3 | 4,4 | 4,4 | 4,4 | 4 | 4,4 |

•all of the above isolated from barley seed of above cultivars
•obtained from Berne Jones (USDA, Madison)
•ref: Ozaki et al., J Biochem 87: 549-555

| | ALO | AFL | FGR | FMO | SSC | STR |
|---|---|---|---|---|---|---|
| Beta purothionin (20 uM = 100 ug/ml) •KSCCKSTLGRNCYNLCRARGAQKLCANVCRCKLTSGLSCPKDFPK | 4,4 | 2,2 | 4,4 | 4,4 | 4,4 | 4,4 |

•obtained from Berne Jones (USDA, Madison)
•isolated from wheat seed
•ref: Stuart and Harris, J. of Ag. and Food Chem., Mar 1942, 19: 288-300

| | ALO | AFL | FGR | FMO | SSC | STR |
|---|---|---|---|---|---|---|
| Stinging nettle lectin (10 uM = 85 ug/ml) | 0,0 | 1,1 | 0,0 | 0,0 | 2,2 | 1,1 |

•obtained from Willy Peumans (Belgium)
•isolated from *Urtica dioica* rhizomes
•relatively small size (8.5 kD)
•high cysteine and tryptophan content
•unusually heat and acid resistant
•specific for N-acetyl-D-glucosamine oligomers
•sequence homology with wheat germ agglutinin
•ref: Broekaert et al., Science Sept 8, 1989, 245, 1100

| | ALO | AFL | FGR | FMO | SSC | STR |
|---|---|---|---|---|---|---|
| Crotamine (30 uM = 137 ug/ml) | 2,2.5 | 1,0 | 2,2.5 | 0,0 | 3,3 | 4,4 |

•isolated from *Crotalus durissus terrificus*
•from Calbiochem (233615)
•snake venom toxin--basic protein of low
toxicity which produces skeletal muscle spasms
that lead to spastic paralysis of peripheral origin
•99% pure
•ref: Tu, A. 1977. Venoms: Chemistry and
Molecular Biology. New York: Wiley Intersciences

EP 0 502 718 A1

Table 5

| | ALO | AFL | FGR | FMO | SSC | STR |
|---|---|---|---|---|---|---|
| Melittin (10.5 uM = 30 ug/ml)<br>•from Sigma (M2272)<br>•isolated from bee venom<br>•~85% peptide by HPLC<br>•Chemical Abstract Survey #37231-28-0 | 4,4 | 1.5,0 | 4,4 | 4,4 | 4,4 | 4,4 |
| Eosinophil major basic protein (9.2 uM = 120 ug/ml)<br>•eight half-cysteines (several are free)<br>•extremely subject to oxidation at neutral-alkaline pH<br>•ref:  Barker, 1988, JEM, 168:1493 | 3,3 | 0,0 | 3,3 | 1,1 | 0,1 | 2,2.5 |
| Eosinophil cationic protein (3 uM = 48 ug/ml)<br>•highly basic (pI >11, 21 Arg)<br>•RNAse activity; neurotoxin and helminthotoxin<br>•ref:  Olsson, 1986, Blood, 67:498 | 2.5,2.5 | 1,1 | 3.5,3.5 | 4,4 | 0,1 | 1,0 |
| Eosinophil peroxidase (21 uM = 400 ug/ml)<br><br>•above three proteins isolated from human leukocytes<br>•all obtained from Gleich (Mayo) | 2,2.5 | 0,0 | 1,1 | 3,3 | 0,0 | 0,0 |

EP 0 502 718 A1

## Table 6

**PARTIALLY PURIFIED PROTEINS**

|  | ALO | AFL | FGR | FMO | SSC | STR |
|---|---|---|---|---|---|---|
| Lyticase (100 ug/ml) | 0,0 | 0,0 | 1.5,1.5 | 1,1 | 0 | 3,4 |
| •from *Arthrobacter luteus* | 0,0 | 0,0 | 2.5,2 | 0,0 | | 3,3.5 |
| •hydrolyzes glucose polymers with ß-1,3 linkages | | | | | | |
| •20% protein, balance potassium phosphate buffer salts | | | | | | |
| •Chemical Abstract Survey #37340-57-1 | | | | | | |
| | | | | | | |
| Penicillinase (300 ug/ml) | 1.5,1.5 | 0,0 | 2,2 | 0,0 | 0,0 | 0,0 |
| •from *Bacillus cereus* | 0,0 | 0,0 | 2.5,2.5 | 0,0 | 0,0 | |
| •ß-lactamase I and II (cephalosporinase) | | | | | | |
| •50% protein, balance primarily buffer and zinc salts | | | | | | |
| •ref: Levy, Nature, 166, 740 (1950) and | | | | | | |
| Pollock and Torriani, Compt. Rend. Acad. Sci. (Paris), 237, 276 (1953) | | | | | | |
| | | | | | | |
| Cytohelicase (1.0 mg/ml) | 4,4 | 4,4 | 4,4 | 4,4 | 4,4 | 4,4 |
| •from *Helix pomatia* | 4,4 | 4,4 | 4,4 | 4,4 | 4,4 | |
| •contains laminarinase activity, but laminarinase itself not active against these fungi | | | | | | |
| •75% protein | | | | | | |
| •ref: Courtois J, Troite de Biochimie Biochimie Generale, Masson and Cie, Ed., 1964, Vol 2 | | | | | | |

Key:
ALO = *Alternaria longipes*
AFL = *Aspergillus flavus*
FGR = *Fusarium graminearum*
FMO = *Fusarium moniliforme*
SSC = *Sclerotinia sclerotiorum*
STR = *Sclerotinia trifoliorum*

EP 0 502 718 A1

Plates can be incubated another 16-10 hours and read spectrophotometrically to determine the turbidity of each well. Turbidity is a measure of the fungal mass per well. The microtiter plates are read on a plate reader at 600 nm and the percent inhibition relative to control is calculated. Four to eight repetitions are needed to accurately determine the percent inhibition with eight repetitions being optimal. Statistical analysis is used to determine whether the proteins significantly inhibit fungi relative to the control.

The above described experiment is then repeated to confirm antifungal activity.

Proteins which exhibit moderate to strong activity against one or more fungi in the first screening process are advanced to a second screening process. The second screening process is designed to determine the lowest concentration of protein that shows inhibitory activity. The screening method is identical to the initial screening except that six serial, three fold dilutions are made of each protein giving approximately 1000X range of concentrations. For example, a protein being screened at $30\mu M$ in the first screening process would be screened at $27\mu M$, $9\mu M$, $3 \mu M$, $1\mu M$, and $0.3\mu M$ in the second screening.

Approximately 90 µl of a protein dilution are put in each well of a microtiter plate. The aliquot of spores or hyphal fragments are added at the same concentrations used in the first screening process.

The same scoring method used in the first screening process is used in the second screening. The experiment is repeated to give an independent verification of activity. The results are recorded as Minimal Effective Concentrations or MEC's. Table 7 gives the results of the Level II screening.

Table 7

Level II results
Data represent minimal effective concentration (MEC), which is the lowest concentration to attain
a score of "2" or greater on a 0-4 scale.

| | ALO | AFL | FGR | FMO | SSC | STR |
|---|---|---|---|---|---|---|
| **Synthetic peptides** | | | | | | |
| Magainin 2 | 27 uM | | 9 uM | | | |
| Magainin A | 27 uM | | 3 uM | 9 uM | 27 uM | |
| Magainin G | 27 uM | | 3 uM | 9 uM | | |
| Poly-L-arginine | 0.9 uM | | 0.3 uM | | 0.9 uM | 0.9 uM |
| Poly-L-histidine 12.8 kD | 2.7 uM | | 2.7 uM | 2.7 uM | 2.7 uM | 2.7 uM |
| Poly-L-histidine 19 kD | 0.9 uM | 2.7 uM | 0.9 uM | 0.9 uM | 2.7 uM | 2.7 uM |
| Mastoparan | 9 uM | | 3 uM | 9 uM | 9 uM | 9 uM |
| Kassinin | | 90 uM | | | | |
| Adrenocorticotropic hormone 1-17 | | | 9 uM | | | |
| Adrenocorticotropic hormone 1-24 | | | 4.5 uM | | | |
| | | | | | | |
| **Purified proteins and enzymes** | | | | | | |
| Citrate synthase | | | 10 ug/ml | | | |
| Defensin 1 | 3 uM | 3 uM | 3 uM | 3 uM | 3 uM | 3 uM |
| Cathepsin G | 2.7 uM | | 0.9 uM | 0.9 uM | | |
| Lysozyme | | | | 2.7 uM | | |
| alpha-Hordothionin | 3 uM | 9 uM | 3 uM | 9 uM | 27 uM | 9 uM |
| ß-Hordothionin (Bomi) | | | | | 18 uM | |
| ß-Hordothionin (Morex) | | 18 uM | 2 uM | 2 uM | 18 uM | 6 uM |
| ß-Purothionin | 2 uM | | 2 uM | 6 uM | 6 uM | 6 uM |
| Crotamine | | | | | 27 uM | 27 uM |
| Melittin | 3 uM | | 1 uM | 3 uM | 3 uM | 9 uM |
| Eosinophil major basic protein | 8 uM | | 8 uM | | | |
| Eosinophil cationic protein | | | 0.9 uM | 2.7 uM | | |
| Eosinophil peroxidase | | | | 19 uM | | |
| | | | | | | |
| **Partially purified proteins** | | | | | | |
| Lyticase | | | | | 10 ug/ml | 10 ug/ml |
| Penicillinase | 270 ug/ml | | 270 ug/ml | | | |
| Cytohelicase | 100 ug/ml | 100 ug/ml | 100 ug/ml | 100 ug/ml | 300 ug/ml | 100 ug/ml |

Key:  ALO = *Alternaria longipes*
AFL = *Aspergillus flavus*
FGR = *Fusarium graminearum*
FMO = *Fusarium moniliforme*
SSC = *Sclerotinia sclerotiorum*
STR = *Sclerotinia trifoliorum*

Preferred proteins for killing or inhibiting the pathogen <u>Fusarium</u> <u>graminearum</u> are: Magainin-2, Magainin-A, Magainin-G, poly-L-Arginine HCl, poly-L-histidine, poly-L-Lysine HBr, poly-L-Lysine HCl, poly-D-Lysine, poly-D-Lysine HBr, Mastoparan, Kassinin, Pro-His-Pro-Phe-His-Phe-Phe-Val-Tyr-Lys, adrenocorticotropic hormone 1-17, Adrenocorticotropic hormone 1-24, Citrate synthase, Defensin NP1, Cathepsin G, Lysozyme, α-hordothionin, β-hordothionin, β-purothionin, Crotamine, Melittin, Eosinophil major basic protein, Eosinophil cationic protein, and Eosinophil peroxidase.

Preferred proteins for killing or inhibiting the pathogen <u>Fusarium</u> <u>moniliforme</u> are: Magainin-2, Magainin-A, Magainin-G, poly-L-Arginine HCl, poly-L-histidine, poly-L-Lysine HBr, poly-L-Lysine HCl, poly-D-Lysine, poly-D-Lysine HBr, Mastoparan, Kassinin, Pro-His-Pro-Phe-His-Phe-Phe-Val-Tyr-Lys, adrenocorticotropic hormone 1-17, Adrenocorticotropic hormone 1-24, Citrate synthase, Defensin NP1, Cathepsin G, Lysozyme, α-hordothionin, β-hordothionin, β-purothionin, Melittin, Eosinophil major basic protein, Eosinophil cationic protein, and Eosinophil peroxidase.

Preferred proteins for killing or inhibiting the pathogen <u>Diplodia</u> <u>maydis</u> are: Magainin-2, Magainin-A, Magainin-G, poly-L-Arginine HCl, poly-L-histidine, poly-L-Lysine HBr, poly-L-Lysine HCl, poly-D-Lysine, poly-D-Lysine HBr, Defensin NP1, α-hordothionin, β-purothionin and Melittin.

Preferred proteins for killing or inhibiting the pathogen <u>Colletototrichum</u> <u>graminicola</u> are: Magainin-A, Magainin-G, poly-L-Arginine HCl, poly-L-histidine, poly-L-Lysine HBr, poly-L-Lysine HCl, poly-D-Lysine, poly-D-Lysine HBr, Defensin NP1, α-hordothionin, β-purothionin and Melittin.

Preferred proteins for killing or inhibiting the pathogen <u>Verticillium</u> <u>albo-atrum</u> are: Magainin-2, Magainin-A, Magainin-G, poly-L-Arginine HCl, poly-L-histidine, poly-L-Lysine HBr, poly-L-Lysine HCl, poly-D-Lysine, poly-D-Lysine HBr, Defensin NP1, α-hordothionin, β-purothionin and Melittin.

Preferred proteins for killing or inhibiting the pathogen <u>Phytophthora</u> <u>megaspermae</u> <u>f.sp.</u> <u>glycinea</u> are: Magainin-2, poly-L-Lysine HBr, Defensin NP1, β-purothionin and Melittin.

Preferred proteins for killing or inhibiting the pathogen <u>Macrophomina</u> <u>phaseolina</u> are: poly-L-histidine, poly-D-Lysine, poly-D-Lysine HBr, Defensin NP1, α-hordothionin and β-purothionin.

Preferred proteins for killing or inhibiting the pathogen <u>Diaporthe</u> <u>phaseolorum</u> <u>caulivora</u> are: Defensin NP1, α-hordothionin and β-purothionin.

Preferred proteins for killing or inhibiting the pathogen <u>Sclerotinia</u> <u>sclerotiorum</u> are: Magainin-A, Magainin-G, poly-L-Arginine HCl, poly-L-histidine, poly-L-Lysine HBr, poly-L-Lysine HCl, poly-D-Lysine, poly-D-Lysine HBr, Mastoparan, Defensin NP1, Cathepsin G, Lysozyme, α-hordothionin, β-hordothionin, β-purothionin, stinging nettle lectin, Crotamine, Melittin, Eosinophil major basic protein and Eosinophil cationic protein.

Preferred proteins for killing or inhibiting the pathogen <u>Sclerotinia</u> <u>trifoliorum</u> are: Magainin-A, poly-L-Arginine HCl, poly-L-histidine, poly-L-Lysine HBr, poly-L-Lysine HCl, poly-D-Lysine, poly-D-Lysine HBr, Mastoparan, Defensin NP1, Cathepsin G, Lysozyme, α-hordothionin, β-hordothionin, β-purothionin, Stinging nettle lectin, Crotamine, Melittin, Eosinophil major basic protein and Eosinophil cationic protein.

Preferred proteins for killing or inhibiting the pathogen <u>Aspergillus</u> <u>flavus</u> are: Magainin-2, Magainin-A, Magainin-G, poly-L-Arginine HCl, poly-L-histidine, poly-L-Lysine HBr, poly-L-Lysine HCl, poly-D-Lysine, poly-D-Lysine HBr, Mastoparan, Kassinin, Defensin NP1, Cathepsin G, Lysozyme, α-hordothionin, β-hordothionin, β-purothionin, Stinging nettle lectin, Crotamine, Melittin and Eosinophil cationic protein.

Preferred proteins for killing or inhibiting the pathogen <u>Alternaria</u> <u>longipes</u> are: Magainin-2, Magainin-A, Magainin-G, poly-L-Arginine HCl, poly-L-histidine, poly-L-Lysine HBr, poly-L-Lysine HCl, poly-D-Lysine, poly-D-Lysine HBr, Mastoparan, Pro-His-Pro-Phe-His-Phe-Phe-Val-Tyr-Lys, adrenocorticotropic hormone 1-17, Adrenocorticotropic hormone 1-24, Citrate synthase, Defensin NP1, Cathepsin G, Lysozyme, α-hordothionin, β-hordothionin, β-purothionin, Crotamine, Melittin, Eosinophil major basic protein, Eosinophil cationic protein, and Eosinophil peroxidase.

Example 2 - Protein Synthesis Methods

Synthetic Peptides:

The synthetic peptides Magainin-2, Magainin-A, Magainin-G, Poly-L-arginine, Poly-L-histidine, Mastoparan, Kassinin, Pro-His-Pro-Phe-His-Phe-Phe-Val-Tyr-Lys, and Adrenocorticotropic hormone 1-17, Adrenocorticotropic hormone 1-24 can be synthesized with a peptide synthesizer using solid phase synthesis and chemical synthesis methods known to the arts. The amino acid sequence is programmed into a peptide synthesizer (Applied Biosystems model 431A) and 100 mg of peptide is obtained. The peptide is purified using reverse phase high pressure liquid chromatography (RP HPLC).

To verify the activity of the synthetic peptide, the activity of the major peak from reverse Phase HPLC is tested for antifungal activity.

To obtain a gene for a synthetic peptide, the optimum codon usage is first determined. Amino acid sequences of proteins from the species being transformed are extracted from GenBank and analyzed. Optimal codons are chosen, based on the average frequency of codons in the proteins. Thus the optimal DNA sequence coding for the desired peptide is determined. An ATG start codon is added to the beginning of the DNA sequence if it is not already present. In order to be able to clone the gene into a plant expression vector, suitable restriction sites are added to either end of the DNA sequence. Then a DNA synthesizer is used to synthesize the oligonucleotide (DNA sequence) and its complement. Oligonucleotides are annealed and cloned into plant expression vectors using techniques known to the art.

Purified Proteins & Enzymes:

The purified proteins and enzymes Citrate Synthase, Defensin 1, Cathepsin G, Lysozyme, α-Hordothionin, β-Hordothionin, β-Purothionin, Stinging Nettle Lectin, Crotamine, Melittin, Eosinophil Major Basic Protein, Eosinophil cationic Protein and Eosinophil Peroxidase are isolated and cloned by the following methods.

Proteins and enzymes are isolated from biological material indicated in the following Table 1 and the references cited. Various purification techniques known to the art are used to purify the proteins and enzymes.

The antifungal activity of purified proteins and enzymes is verified by associating antifungal activity with a single band on a polyacrylamide gel or a single peak from Reverse Phase HPLC.

To clone the gene for a purified protein, any one of the following strategies may be employed:

a. The protein (or proteolytic products) is sequenced. A degenerate oligonucleotide probe is designed based on the coding sequence of from 6-20 amino acids of the sequence.

b. Antibodies to the protein may be used to screen an expression library. This technique is used if it is easier to raise antibodies to the protein of interest than to sequence the protein. Using molecular biology techniques well known to the art, a lambda phage expression library is constructed with cDNA from the source of the protein. This library contains cDNA inserts in all possible reading frames inserted into a β-galactosidase open reading frame. Using procedures known to the art, the library can be screened for epitopes that react to the antibodies. The clones are then sequenced and the protein size is verified. The gene can then be inserted into a plant expression vector using techniques known to the art and described in the background to the invention.

c. Polymerase chain reaction (PCR) is another well known technique for cloning genes. This procedure uses DNA primers based on two separate regions of the protein for which the sequence is known. (Codons may or may not be degenerate.) The PCR procedure is used to amplify a specific DNA fragment between the primers. That fragment can be purified and used as a very specific probe. A genomic, or, preferably, cDNA library is screened using standard cloning techniques. The clone identified by the oligonucleotide is sequenced to verify an open reading frame. The size of the expected protein is verified against the size of the purified protein. Using in vitro mutagenesis, restriction sites for cloning are generated at the 3′ and 5′ ends of the gene. The gene is then inserted into a plant expression vector using cloning techniques well known to the art.

Partially Purified Protein Extracts:

The proteins Lyticase, Penicillinase and Cytohelicase have not been purified to homogeneity. To identify the element responsible for the antifungal activity the protein extract may be analyzed by polyacrylamide gel electrophoresis or by Reverse Phase HPLC and all bands or fractions tested for antifungal activity. Those fractions associated with antifungal activity can be further purified until a single band is obtained. The gene may then be cloned using the procedures described herein for purified proteins.

All percentages herein are by weight, except as otherwise indicated. The term "microorganisms" as used herein includes bacteria and fungi. The terms "bacteria" and "bacterial infection" are used herein in their usual context. By "fungi" herein are meant the higher protists, including the phycomycetes, the ascomycetes and basidiomycetes, as well as other protista commonly referred to as "yeasts" or "molds".

## Claims

1. A method for killing or inhibiting the growth of pathogenic microorganisms which are susceptible to the proteins: Adrenocorticotropic Hormone, preferably Adrenocorticotropic Hormone 1-17 or 1-24, Lyticase, Penicillinase, Kassinin, Mastorparan, Melittin, Pro-His-Pro-Phe-His-Phe-Phe-Val-Tyr-Lys, Synthase, or Crotamine comprising introducing into the environment of the pathogenic microorganisms an antimicrobial amount of such a protein.

2. A method according to claim 1 wherein the pathogenic microorganism is <u>Fusarium</u> <u>graminearum</u> <u>Fusarium</u> <u>moniliforme</u>, <u>Diplodia</u> <u>maydis</u>, <u>Colletototrichum</u> <u>graminicola</u>, <u>Verticillium</u> <u>albo-atrum</u>, <u>Phytophthora</u> <u>megas-permae</u> <u>f.sp.</u> <u>glycinea</u>, <u>Macrophomina</u> <u>phaseolina</u>, <u>Diaporthe</u> <u>phaseolorum</u> <u>caulivora</u>, <u>Sclerotinia</u> <u>sclerotiorum</u>, <u>Sclerotinia</u> <u>trifoliorum</u>, <u>Aspergillus</u> <u>flavus</u>, or <u>Alternaria</u> <u>longipes</u>, and wherein the protein may also be selected from: Defensin NP1, Magainin-2, Magainin-A, Magainin-G, $\alpha$-Hordothionin, $\beta$-Hordothio-nin, $\beta$-Purothionin, poly-L-Lysine HBr, poly-L-Lysine HCl, poly-D-Lysine, poly-D-Lysine HBr, poly-L-Arginine HCl, poly-L-Histidine, Cathepsin G, Lysozyme, Eosinophil Major Basic Protein, Eosinophil Cationic Protein, Eosinophil peroxidase, Stinging Nettle Lectin, citrate synthase or cytohelicase.

3. A method according to claim 1 or 2 wherein the environment of the pathogen is the locus of a plant.

4. A method according to any one of claims 1 to 3 wherein the environment of the pathogen is the tissues of a plant.

5. A method according to claim 4 wherein the protein is provided to the tissues of the plant by a nucleic acid sequence which has been inserted into the genome of the plant, said sequence coding for the protein and in proper reading frame relative to transcription initiator and promoter sequences active in the plant, whereby the protein is expressed at levels which provide an antimicrobial amount of the protein in the tissues of the plant.

6. A method according to claim 5 wherein the protein is not native to the plant.

7. A method according to any one of claims 3 to 6 wherein the plant is a monocotyledonous species selected from wheat, rice, and sorghum.

8. A method according to any one of claims 5 to 7 wherein the sequence has been inserted by the steps of:
a) culturing cells or tissues from the plant;
b) introducing into the cells of the cell or tissue culture at least one copy of an expression cassette comprising a sequence coding for the protein; and
c) regenerating protected whole plants from the cell or tissue culture

9. A method according to claim 8 which comprises the further step of sexually or clonally reproducing the whole plant in such manner that at least one copy of the sequence provided by the expression cassette is present in the cells of progeny of the reproduction.

10. A method according to claim 8 or 9 in which the expression cassette is introduced into the cells by electroporation.

11. A method according to claim 8 or 9 in which the expression cassette is introduced into the cells by microparticle bombardment.

12. A method according to claim 8 or 9 in which the expression cassette is introduced into the cells by microinjection.

13. A method according to claim 8 or 9 for providing resistance to microorganisms in <u>Agrobacterium</u> <u>tumefa-ciens</u> susceptible dicotyledonous plants in which the expression cassette is introduced into the cells by infecting the cells with <u>Agrobacterium</u> <u>tumefaciens</u>, a plasmid of which has been modified to include the expression cassette.

14. A method of imparting resistance to diseases caused by a pathogen as recited in claim 2 to plants of a taxon susceptible to those diseases, comprising the steps of:
a) selecting a fertile, disease resistant plant prepared by the method of any one of claims 8 to 13 from a sexually compatible plant;
b) sexually crossing the disease resistant plant with a plant from the disease susceptible taxon;
c) recovering reproductive material from the progeny of the cross; and
d) growing protected plants from the reproductive material.

15. A method according to any one of claims 8 to 13 for imparting resistance to diseases caused a pathogen in a taxon consisting of substantially homozygous plants, which comprises the further steps of repetitively:

a) backcrossing the disease resistant progeny with substantially homozygous, disease susceptible plants from the taxon; and

b) selecting for expression of disease resistance and antimicrobial activity along with the other desired characteristics of the susceptible taxon from among the progeny of the backcross, until the desired percentage of the characteristics of the susceptible taxon are present in the progeny along with disease resistance and antimicrobial activity.

16. A DNA clone from the genome of a plant which codes substantially solely for a protein selected from the group of claim 1.

17. An expression cassette comprising a DNA clone according to claim 16, operably linked to plant regulatory sequences which cause the expression of the DNA clone in plant cells.

18. An expression cassette comprising a DNA clone according to claim 16 operably linked to bacterial expression regulatory sequences which cause the expression of the DNA clone in bacterial cells.

19. Bacterial cells containing as a foreign plasmid at least one copy of an expression cassette according to claim 18.

20. Transformed plant cells containing as foreign DNA at least one copy of the DNA sequence of an expression cassette according to claim 17.

21. Transformed cells according to claim 20, further characterized in being cells of a monocotyledonous species, preferably maize, sorghum, wheat or rice cells.

22. Transformed cells according to claim 20, further characterized in being cells of a dicotyledonous species, preferably soybean, alfalfa, tobacco or tomato cells.

23. A maize cell or tissue culture comprising cells according to claim 21.

24. A transformed maize plant, the cells of which contain as foreign DNA at least one copy of the DNA sequence of an expression cassette according to claim 17.

25. A antimicrobial composition, comprising a antimicrobial amount of a protein according to claim 1 in a non-toxic vehicle.

26. A composition according to claim 25 wherein the vehicle is adapted for systemic administration to a susceptible organism.

27. A pharmaceutical composition in unit dosage form having antifungal activity which comprises a safe and effective amount of a protein according to claim 1.

28. A protein as defined in claim 1 for use in a method of antifungal treatment of the human or animal body.

29. Use of a protein as defined in claim 1 for the manufacture of a medicament for the treatment of fungal infections of the human or animal body.

European Patent
Office

**EUROPEAN SEARCH REPORT**

Application Number

| | DOCUMENTS CONSIDERED TO BE RELEVANT | | EP 92301868.3 |
|---|---|---|---|

| Category | Citation of document with indication, where appropriate, of relevant passages | Relevant to claim | CLASSIFICATION OF THE APPLICATION (Int. Cl.5) |
|---|---|---|---|
| X | EP - A - 0 254 419 (CHISSO CORPORATION) * Abstract; claims * -- | 1,2 | A 01 N 37/46 A 01 N 37/44 A 01 N 63/00 A 01 N 65/00 |
| P,X | EP - A - 0 427 529 (PIONEER HI-BRED INTERNATIONAL INC.) * Abstract; claims * -- | 1-25 | C 12 N 15/00 C 12 N 15/52 |
| A | DE - A - 3 500 823 (MEYER-GLAUNER) * Abstract * -- | 1 | |
| A | EP - A - 0 348 348 (CIBA-GEIGY AG) * Abstract; claims * -- | 1-25 | |
| D,A | EP - A - 0 186 425 (ELI LILLY AND COMPANY) * Abstract; claims * -- | 1-25 | |
| D,A | EP - A - 0 204 590 (INSTITUT NATIONAL DE LA RECHERCHE AGRONOMIQUE (INRA)) * Abstract; claims * -- | 1-25 | TECHNICAL FIELDS SEARCHED (Int. Cl.5) A 01 N C 12 N |
| D,A | WO - A - 88/07 087 (AMERICAN BIOGENETIC SCIENCES) * Abstract * ---- | 1-25 | |

The present search report has been drawn up for all claims

| Place of search | Date of completion of the search | Examiner |
|---|---|---|
| VIENNA | 03-06-1992 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)